# EUROPEAN PATENT SPECIFICATION

(11) **EP 1 547 523 B1**
(45) Date of publication and mention of the grant of the patent: **23.07.2008**
(21) Application number: 03258159.7
(22) Date of filing: 24.12.2003
(51) Int. Cl.: A61B 5/08

(54) **Method and apparatus for synchronizing respiratory gas measurements**
Verfahren und Vorrichtung zur Synchronisation der Atemgasmessungen
Procédé et dispositif pour synchroniser les mesures de gaz respiratoire

(43) Date of publication of application: 29.06.2005
(73) Proprietor: GE Healthcare Finland Oy, 00510 Helsinki (FI)
(72) Inventor: Coffeng, René, 00750 Helsinki (FI); Heinonen, Erkki Paavo Olavi, 00750 Helsinki (FI)
(74) Representative: Charlton, Peter John

(56) References cited:
- EP-A- 0 392 503
- US-A- 4 090 078
- US-A- 4 368 740
- US-A- 5 069 220
- PATENT ABSTRACTS OF JAPAN vol. 012, no. 042 (P-663), 6 February 1988 (1988-02-06) & JP 62 188969 A (TORAY IND INC), 18 August 1987 (1987-08-18)

## Description

The present invention relates to a method and apparatus that may be used to synchronize or coordinate disparate measurements taken from the respiratory gases of a subject. The synchronization so provided insures that the determination of gas exchange parameters obtained using the measurements accurately reflect gas exchange by the subject. The method and apparatus more generally relate to determining the transit time of a breathing gas sample in a sampling line for a remote gas concentration sensor. The method and apparatus may also be used to determine the volume of the sampling line.

During respiration, gas exchange occurs in the lungs between the breathing gases of a subject and the blood. This gas exchange occurs by diffusion across thin membranes between gas filled alveoli in the lungs on one side of the membranes and blood filled capillaries of the circulatory system on the other side of the membranes. Differences in the partial pressures of gases in the alveoli and gases dissolved in the blood drive the diffusion. Venous blood entering the lung from the pulmonary artery has an increased or enriched level of carbon dioxide (CO₂) as a result of metabolism occurring in the organs and muscles of the body. Metabolism consumes oxygen (O₂) from the blood so that venous blood is also characterized as having a reduced or depleted O₂ content.

When the blood passes through the pulmonary capillaries of the lung, the gas diffusion occurring across the membranes in the lung equilibrates the blood gas partial pressures with the alveolar gas partial pressures so that CO₂ is transferred from the blood to the alveolar gas and O₂ is transferred from the alveolar gas to the blood.

During inspiration, the lungs are supplied with additional gas over the volume of gas in the lungs at the end of expiration. The additional gas is typically atmospheric air containing 0.03 percent CO₂ and 20.9 percent O₂. Alternatively, if the subject breathes with the aid of a mechanical ventilator, the content of the inspired breathing gases can be determined by the gases supplied to the ventilator and the mixing occurring therein. In this case the CO₂ content of the breathing gases is typically negligibly small and the O₂ concentration varies from 25 to 100 percent.

The gases supplied to the lungs during inspiration dilute the alveolar gases existing in the lung and the breathing gases subsequently expired by the subject comprise a mixture of the inspired gases and alveolar gases.

CO₂ production per breath of a subject is measured as the difference between the volume of CO₂ in the expired breathing gases and the volume of CO₂ in the inspired breathing gases. Correspondingly, O₂ consumption per breath is measured as the difference in the inspired and expired O₂ volumes of the breathing gases. Gas exchange parameters, such as CO₂ production (VCO₂, mL/min) and O₂ consumption (VO₂, mL/min), are the minute sum of the CO₂ production and O₂ consumption occurring in the individual breaths.

In addition to such measurements, other gases are exchanged with ventilation for other purposes, such as to put a patient into anesthesia, for therapy, or to make lung function measurements. Gases involved in these processes include e.g. nitrous oxide (N₂O), volatile anesthetic gases (e.g. halothane, enflurane, isoflurane, sevoflurane, desflurane), helium, carbon monoxide (CO), nitric oxide (NO), and sulfur hexafluoride (SF₆).

The volume of a particular gas may be determined by obtaining the flow rate of the particular gas and integrating the flow rate with respect to time, e.g. over a breath. The flow rate of the particular gas is obtained by measuring the flow rate of the breathing gases of the subject and multiplying that flow rate by the gas concentration.

Breathing gas flow is typically measured with a sensor that provides a signal having a first polarity for gas flow in one direction and the opposite polarity for gas flow in the opposite direction. Such a sensor may, for example, incorporate a flow restricting element and a pressure sensing element to detect pressure differences caused by gas flow through the restriction. A Fleish pneumotachograph is a well known example of such a sensor. Another suitable sensor is that shown in U.S. Patent No. 5,088,332 of the Instrumentarium Corp. of Helsinki, Finland.

Gas concentration sensors can be divided into two classes: mainstream sensors and side-stream sensors. In a mainstream gas concentration sensor, the entire gas flow passes through the sensor element, whereas in a side-stream gas concentration sensor, only a small sample flow (typically less than 200 mL/min) is withdrawn from the main gas flow to a sensor element located out of the main gas flow. The point at which the gas sample is withdrawn is typically at, or proximate to, the point at which the gas flow sensor is located. A flow generator, such as a pump, is used to establish the sample flow. The gas concentration sensor may typically be a paramagnetic device for O₂ and an infrared absorption device for CO₂.

An advantage of a mainstream gas concentration sensor is that it measures the same breathing gases as are passing through the gas flow sensor. This makes correlation of the flow and concentration measurements used to determine gas volumes relatively easy and the determination of the corresponding gas exchange parameters, such as CO₂ production or O₂ consumption, is relatively accurate. The disadvantages of a mainstream sensor includes its large size. The measurement site is typically near the subject's mouth which is often crowded by other measurement apparatus or therapeutic devices. Also, the sensing component for such a sensor tends to be expensive and there is a risk of damage when it is located at or near the subject.

Side-stream gas concentration sensors avoid the foregoing problems but raise another problem caused by the time delay resulting from the time required to move the sample from the sampling point in the breathing gas passage to the remote measurement site. Thus, before a side-stream gas concentration sensor can be used for gas exchange measurement of the type described above, the measured concentration obtained from the remote side-stream gas concentration sensor must be accurately synchronized with the real-time flow signal obtained from the flow sensor in breathing gas passage of the subject so the flow and concentration sensings reflect those from the same quantity of breathing gases.

To synchronize the breathing gas concentration measurements with the breathing gas flow measurements, the flow measurement has to be delayed by the time delay occurring for the gas concentration measurement. The remote side-stream gas concentration sensor is typically connected to the breathing gas passage for the subject by a tube of about 1 mm internal diameter and 2-3 meters in length. The sampling path may also include components to prevent bacteria and water from entering the sensing apparatus. The amount of the pneumatic time delay of the breathing gas concentration sensor measurement depends on two variables; the gas flow rate in the sampling path and the sampling path volume. Both of these factors may and, usually will, vary in the course of gas exchange measurements and even in the course of a single breath. The flow rate varies in response to changes in sampling path flow resistance and the effect created by the sampling flow generator, such as a pump. When the subject is breathing with the aid of a mechanical ventilator, pressures in the breathing gas passage also cause variations in the sampling gas flow rate. Sampling path flow resistance may vary, for example, as a result of moisture condensation that affects both sampling line volume and flow generator efficiency.

Because of the many, and somewhat unpredictable, sources of variation in gas concentration measurement time delay, breathing gas analysis systems using side stream gas concentration sensors have to incorporate an automatic on-line technique to ascertain the amount of time delay in the gas concentration measurement. Such a technique typically utilizes readily identifiable, marked changes in breathing gas flow and gas composition properties. As noted above, the polarity of the signal from the gas flow sensor in the breathing gas passage changes as the gas flow direction changes during the transition from inspiration to expiration, and vice versa. When such a polarity change is detected, a time delay measurement timer in the system is reset and restarted. The switch between inspiration and expiration, and vice versa, is also associated with changes in the gas composition of the breathing gases in the breathing gas passage: i.e. inspiration gases have higher O₂ and lower CO₂ amounts; expiration breathing gases have lower O₂ and higher CO₂ amounts. The detection of a gas composition change in the gas concentration sensor stops the time delay measurement timer and provides an indication of the amount of the time delay.

However, to ensure accurate synchronization between side-stream measured breathing gas concentrations and real-time gas flows in the breathing gas passage, one additional factor must be considered. The sampling point for breathing gas flow and composition will be at some defined point along the reciprocal flow breathing gas passage for the subject. At both ends of the breathing gas passage there will be some volume of dead space that has to pass the sampling point before the breathing gases, the gas concentration of which is to be measured, will pass the sampling point from which the breathing gas sample is drawn. The amount of time required for the breathing gases to pass through the dead space to the sampling point causes an additional delay in the breathing gas concentration measurement and the time delay determined by the measurement timer must be compensated for this additional delay in order to obtain accurate measurement synchronization. The compensation required for movement of the breathing gases through the dead space is often an assumed amount based on measurements of the geometry of the breathing gas passage.

Another approach to achieving accurate synchronization of breathing gas flow measurements and gas concentration measurements is to measure the sampling path volume and sampling path flow rate. For this purpose, the sampling path is provided with an appropriate flow sensor. The sampling path volume can be determined from measurements of the internal diameter and length of the sampling path tubing. Knowing the sampling path volume and the flow in the sampling path, the time when the measured gas sample entered the sampling path from the sampling point in the breathing gas passage can be backwards calculated. This data is then used to put the gas concentration measurement together with the corresponding flow rate for obtaining the desired gas exchange measurement for determining metabolic activity. This method also requires compensation for dead space volumes in the breathing gases passage and with either method, when the geometry of the breathing gases passage is changed, for example, by changing the location of the sampling point or changing from an adult sized breathing gas passage to a pediatric sized breathing passage, this compensation will no longer be valid.

Still further, the amount of the additional compensation required to synchronize flow and concentration sensing may also depend on the settings applied to a mechanical ventilator. Side-stream gas concentration measurement suctions gas from the sampling point in the breathing gas passage to the concentration sensor by a gas flow generator that, as noted above, is typically a pump. If the mechanical ventilator provides a prolonged expiratory pause during which there is no expiratory breathing gas flow, sampling gas is drawn into the side-stream sampling path by the pump. Inspiratory breathing gas may be mixed with this gas, invalidating the assumptions on which the time delay calculations are based. In extreme cases, the inspiratory gas may enter the sampling path before the flow sensor resets the delay time measurement timer. This may have a major effect on the accuracy of the gas exchange measurements obtained from the breathing gas flow and concentration data.

US-A-5069220 discloses a method for analyzing the concentration of selected constituent gases in the expiratory gas streams from a patient. The method includes the steps of: measuring the flow rate of the expiratory gas stream to provide a flow rate signal; generating a tidal volume signal in response to the flow rate signal; generating a sample control signal in response to the tidal volume signal and the flow rate signal to mark an interval within the end tidal periods of several successive expiratory gas streams; extracting a sample of each of the several successive expiratory gas streams during the marked interval in response to the sample control signal; accumulating each extracted sample in a variable volume reservoir; transferring the accumulated gas samples within the variable volume reservoir into a sample cell in response to the variable volume reaching a predetermined limit; and measuring the concentration of the selected constituent gas in the volume of gas contained in the cell. The method optionally includes the further steps of: measuring the concentration of oxygen in an inspiratory gas stream conducted to the patient; and calculating the patient's oxygen consumption by comparing the measured concentration of oxygen in the gas contained in the sample cell with the measured concentration of oxygen in the inspiratory gas stream.

US-A-4368740 discloses a physiologic analyzer for continuous measurement of a subject's metabolic functions in which signals from ventilatory flow rate sensors and gas analyzers are processed to provide continuous measurement of the subject's ventilatory volume, carbon dioxide production, oxygen consumption, respiratory exchange ratio, and other metabolic functions of interest. An expiratory oxygen concentration sampler is enabled only when oxygen concentration deviates from the inspired value. When enabled, the sampler provides at its output a series of discrete signals proportional to successive instantaneous values of oxygen concentration in expired breath. A similar sampler is used in the processing of the carbon dioxide concentration signals. A respiratory cycle timer measures elapsed time between successive inspirations and provides a breath duration signal which is used for rate computations. Interpretation and analysis of test results is facilitated by displaying all metabolic measurements as a function of oxygen consumption.

According to a first aspect of the invention, there is provided a method for determining the volume of a breathing gas sampling conduit extending between a sampling point in a breathing gas passage for the breathing gases of a subject and a gas concentration sensors remote from the sampling point, the gas concentration sensor measuring the concentration of a given gas in the sample, the breathing gas alternately flowing in a first direction in the breathing gas passage and in a second, opposite direction in the breathing gas passage, the breathing gases exhibiting a first concentration characteristic of the given gas when the breathing gas flow is in the first direction and a second, different concentration characteristic when the breathing gas flow is in the second direction, said method comprising the steps of
reducing the flow of sample breathing gases in the breathing gas sampling conduit when the breathing gases are flowing in the breathing gas passage in a first direction and the breathing gases exhibit the concentration characteristic of that breathing gas flow direction;
allowing the flow direction of the breathing gases in the breathing gas passage to reverse to the second direction and the breathing gas concentration characteristic to change to the gas concentration characteristic for the second flow direction;
resuming the flow of sample breathing gases in the breathing gas sampling conduit and initiating the commencement of a measurement interval;
measuring the breathing gas sample flow rate through the breathing gas sampling conduit;
determining the volume of the breathing gas sampling conduit using the measured flow rate in the time following resumption of the flow of sample breathing gases;
sensing the concentration of the given gas in the breathing gases sample flowing in the breathing gas sampling conduit with the breathing gas concentration sensor; and
terminating the measurement interval upon the gas concentration sensor sensing an alteration in the given gas concentration characteristic from the characteristic of the first breathing gas flow direction.

Preferably, the method is further defined as a method for determining the transit time of a breathing gas sample along the breathing gas sampling conduit, said method comprising the additional steps of initiating the commencement of a transit time timing interval upon the resumption of the flow of sample breathing gases in the breathing gas sampling conduit; and terminating the timing interval upon the gas concentration sensor sensing an alteration in the given gas concentration characteristic from the characteristic of the first breathing gas flow direction to the characteristic of the second breathing gas flow direction, the time interval so defined being an indication of the transit time of the breathing gas sample along the breathing gas sampling conduit extending from the entry of the sampling conduit to the gas concentration sensor.

Further, the method preferably uses the step of using the transit time so measured to synchronize a gas flow measurement obtained at the sampling point with a given gas concentration measurement obtained by the gas concentration sensor.

The invention also provides an apparatus for use with a breathing gas sampling conduit extending between a sampling point in a breathing gas passage for the breathing gases of a subject and a gas concentration sensor remote from the sampling point, the gas concentration sensor measuring the concentration of a given gas in the sample, the breathing gas alternately flowing in a first direction in the breathing gas passage and in a second, opposite direction in the breathing gas passage, the breathing gases exhibiting a first concentration characteristic of the given gas when the breathing gas flow is in the first direction and a second, different concentration characteristic when the breathing gas flow is in the second direction, said apparatus comprising:
a measurement and control means coupled to the gas concentration sensor;
a breathing gas sensor for determining the flow direction of the breathing gases in the breathing gas passage, said breathing gas sensor being coupled to said measurement and control means;
flow control means operable by said measurement and control means for controlling the flow of sample breathing gases in the breathing gas sampling conduit, said measurement and control means operating said flow control means to reduce the flow of sample breathing gases in the sampling conduit when the breathing gases are flowing in the breathing gas passage in a first direction and the breathing gases exhibit the concentration characteristic of that breathing gas flow direction, and to thereafter resume the flow of sample breathing gases in the breathing gas sampling conduit when the flow direction of the breathing gases in the breathing gas passage has reversed to a second direction and the breathing gas concentration characteristic has changed to that of the second direction; and
said measuring and control device having a timer that initiates a measurement interval when the flow of sample breathing gas resumes and terminates the interval when the gas concentration sensor senses an alteration in the given gas concentration characteristic from the characteristic of the first breathing gas flow direction to the characteristic of the second breathing gas flow direction.

The invention thus provides an improved method and apparatus for determining the transit time of a breathing gas sample in the sampling line to a side-stream gas concentration sensor. In application, the present invention may be used to accurately synchronize breathing gas flow and concentration measurements so as to overcome the problems noted above.

The present invention may be used with any mode of ventilation and with any breathing gases passage sampling point geometry.

The invention also provides a technique for accurately determining the gas sampling line volume for a side-stream gas concentration measurement device that is suitable for use in any mode of ventilation of a subject and with any breathing gases passage sampling point geometry. Knowing the breathing gas sample flow rate, the needed synchronization between breathing gas flow and concentration measurements can be determined.

Essentially, in the method and apparatus of the invention, the flow of the sample breathing gases in the breathing gas sampling conduit or line is reduced, preferably stopped, when breathing gases are flowing in a first direction in the breathing gas passage for the subject and the breathing gases exhibit a gas concentration characteristic of that breathing gas flow direction. For example, breathing gas flow may be stopped during expiration when the breathing gases are characterized by the presence of a CO₂ concentration. The flow of sample breathing gases may be controlled by closing a valve in the breathing gas sampling line or by stopping operation of a flow generating pump for the sampling conduit.

Thereafter, the flow direction of the breathing gases in the breathing gas passage for the subject is allowed to reverse to a second direction and the breathing gas concentration characteristic allowed to change to that of the second flow direction. In the present example, the flow direction of breathing gases reverse to that of inspiration, which flow direction is characterized by an absence of a concentration of CO₂.

The flow of sample breathing gases in the breathing gas conduit is then resumed. This starts a boundary or front between breathing gases exhibiting the concentration characteristic of the first flow direction and those exhibiting the concentration characteristic for the second flow direction moving along the sampling conduit from the entry of the sampling conduit toward the gas concentration sensor. A transit time timing interval is initiated when the flow of sample breathing gases is resumed. The CO₂ concentration in the breathing gas sample is sensed by the breathing gas concentration sensor and the timing interval is terminated when the gas concentration sensor senses the alteration from the gas concentration characterizing the first breathing gas flow direction to the gas concentration characterizing the second breathing gas flow direction. The timing interval so defined is an indication of the transit time of the breathing gas sample from the entry of the sampling conduit to the gas concentration sensor. The timing interval so determined may be used to synchronize breathing gas flow measurements and breathing gas concentration measurements to obtain metabolic gas exchange parameters such as CO₂ production of O₂ consumption. By measuring the breathing gas sample flow rate and integrating it over the timing interval, the volume of the sampling conduit may be determined.

Examples of the invention will now be described in detail with reference to the accompanying drawings, in which:
Fig. 1 is a diagram showing apparatus of the present invention suitable for carrying out the method of the present invention;
Figs. 2a and 2b are graphs showing breathing gas flow and CO₂ concentration measurements taken at a common sampling point;
Figs. 3a and 3b are graphs showing breathing gas flow measurements taken at the sampling point and CO₂ concentration measurements taken at a side-stream gas concentration sensor remote from the sampling point; and
Figs. 4a through 4f are graphs illustrating the method of the present invention.

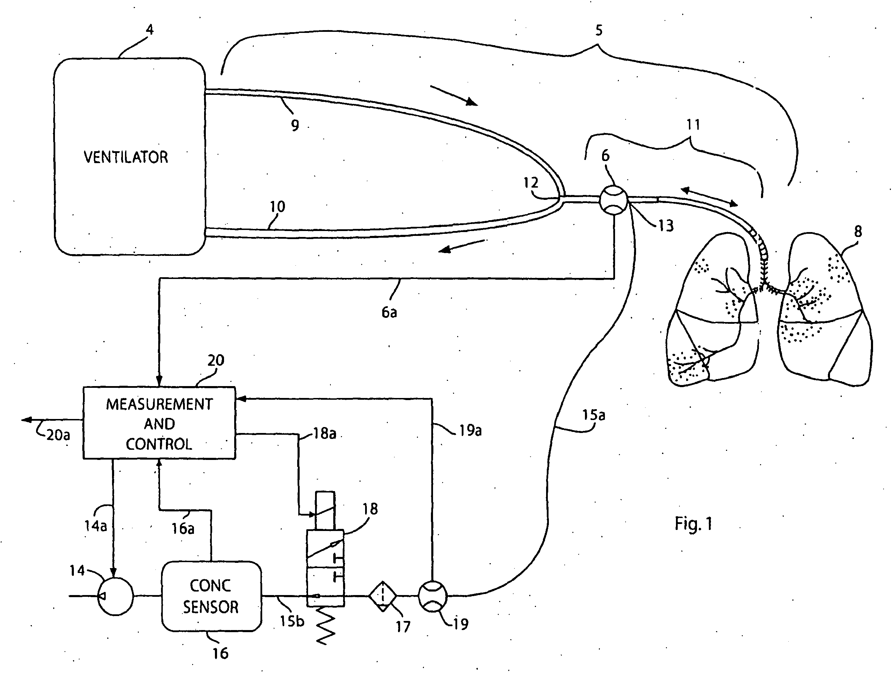

Fig. 1 shows apparatus in which the breathing of a subject is established by ventilator 4. Ventilator 4 may be of conventional construction. Ventilator 4 controls the amount and concentration of breathing gases supplied to breathing circuit 5 and also carries out the removal of gases from the breathing circuit. Breathing circuit 5 includes inspiration limb 9, expiration limb 10, patient limb 11, and wye-piece 12 connecting the inspiration, expiration, and patient limbs. The breathing gas passage comprising patient limb 11 communicates with the subject's lungs 8.

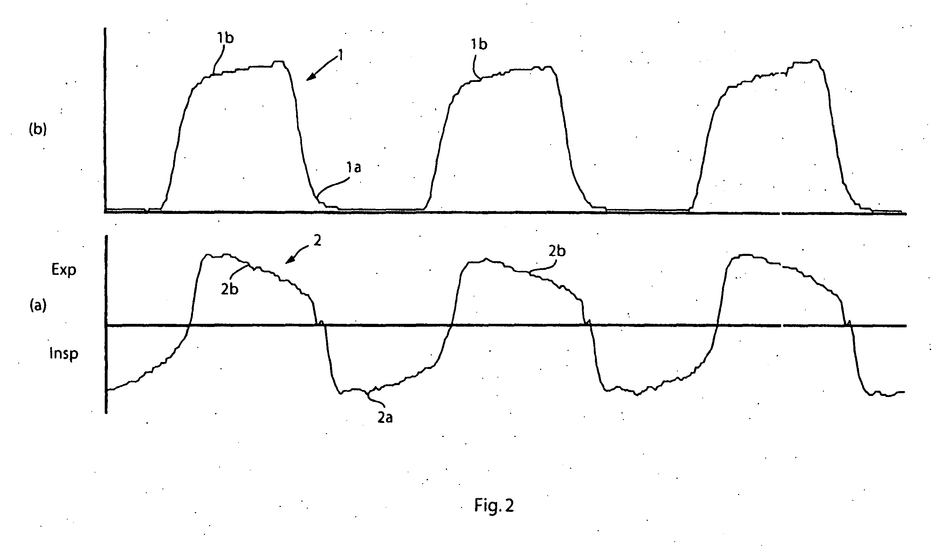

A breathing gas sampling point 13 is located in patient limb 11 for obtaining an indication of breathing gas properties for both the inspiration and expiration of breathing gases. If both the breathing gas flow rate and the concentration of a particular breathing gas, such as CO₂, are measured at sampling point 13, the resulting measurement would be those shown in Fig. 2. Measurements for three breaths are shown, with time as the abscissa. Fig. 2a shows the bidirectional breathing gas flow 2, with flow in one direction during expiration being shown above the abscissa and flow in the opposite direction during inspiration being shown below the abscissa. Fig. 2b shows the corresponding CO₂ measurement 1 with the CO₂ generated by the subject's metabolism being present in the expired breathing gases. If both measurements are taken at the sampling point 13, there will be no time delay between the gas flow measurement 2b and the gas concentration measurement 1a, also as shown by Figs. 2a and 2b.

With side-stream gas concentration measurement, the gas flow and gas concentration are not sensed at a common sampling point. Rather, gas flow is sensed in patient limb 11 at sampling point 13 and gas concentration is sensed remotely from patient limb 11. Thus, as shown in Fig. 1, only gas flow sensor 6 is provided at sampling point 13. While it is advantageous to have a single flow sensor providing breathing gas flow rates for both inspiration and expiration, separate breathing gas flow sensors may be used, if desired. Such breathing gas flow sensors may also be located elsewhere in the breathing circuit or within ventilator 4.

In the side-stream gas concentration measurement with which the present invention is concerned, a breathing gas sample is drawn from sampling point 13 with the aid of flow generator 14 through sampling line or conduit 15a to normally open calibration valve 18. The sampling path continues from calibration valve 18 to gas concentration sensor 16 in line 15b. The gas flow generator may typically be a pump of a known type, such as a piston or membrane pump. Gas sampling path 15a may incorporate sample gas flow rate sensor 19 as well as a means 17 to prevent bacteria, viruses, and/or water from entering gas concentration sensor 16.

Breathing gas flow sensor 6, calibration valve 18, sample flow rate sensor 19, gas concentration sensor 16, and sample gas flow generator 14 are connected to measurement and control device 20 through respective connection lines 6a, 18a, 19a, 16a, and 14a.

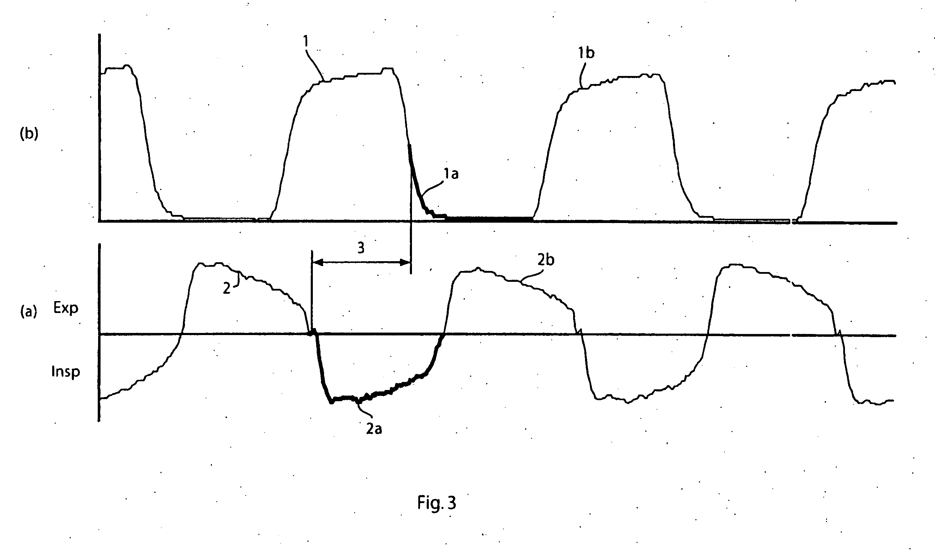

As described above, with conventional side-stream gas measurement apparatus, the CO₂ concentration curve 1, shown in Fig. 3b and obtained from gas concentration sensor 16 is delayed with respect to the breathing gas flow curve 2 obtained from the breathing gas passages for the subject by flow sensor 6 and shown in Fig. 3a. The delay is caused by the transit time delay required for the gas sample to move through the sampling line 15a, 15b from sampling point 13 in the breathing gas passage to gas concentration sensor 16. The gas flow measured in the breathing gas passage for an inspiratory phase of a breath is shown by the thick line 2a in Fig. 3a. The corresponding CO₂ gas concentration measurement is shown by the thick line 1a in Fig. 3b. Measurements for the succeeding expiratory phase of the respiratory cycle are shown with thin lines 1b and 2b, respectively. Corresponding delays occur in the expiratory phase of the respiratory cycle when CO₂ produced by metabolism is present in the breathing gases. The lack of synchronization, or the time delay, between a gas flow measurement 2a, 2b and corresponding gas concentration measurement 1a, 1b is indicated by the time interval 3 in Fig. 3.

As noted above, it is the object of the present invention to accurately determine time delay 3 so that the gas concentration measurement 1a, 1b can be appropriately matched with the respective flow rate measurement 2a, 2b, respectively so as to permit proper measurement of the gas volumes of interest to determine the gas exchange activity of the subject.

While Figs. 1 and 3 show the measurement of CO₂ in exemplary fashion, it will be appreciated that other breathing gases, such as O₂, exhibit an analogous phenomenon.

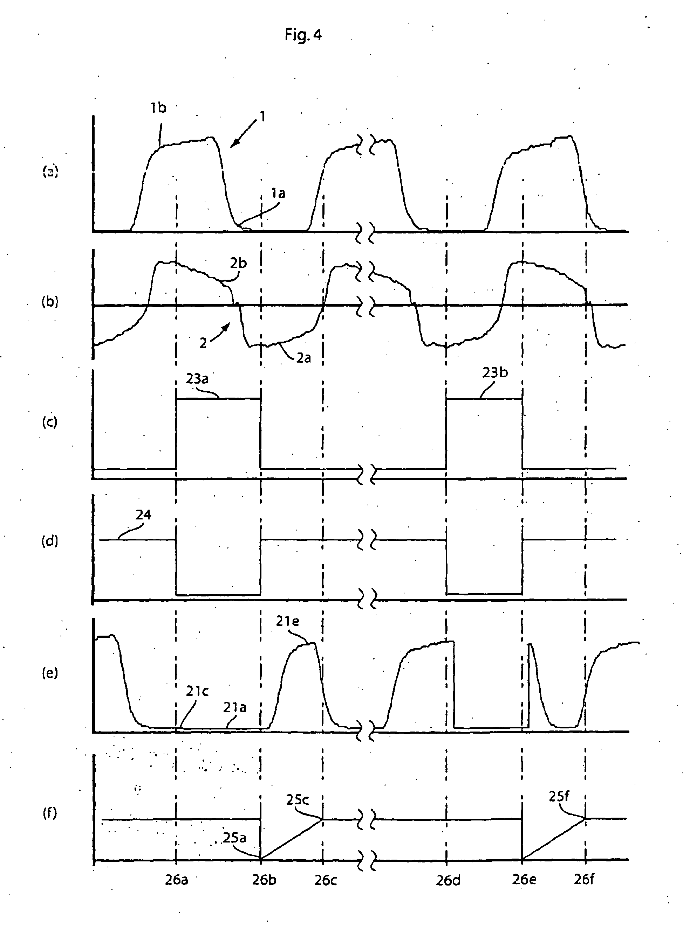

The technique of the present invention using the apparatus of Fig. 1 is presented schematically in Fig. 4. Figs. 4a and 4b show breathing gas CO₂ concentration 1 and breathing gas flow 2 at sampling point 13 in the same manner as in Figs. 2b and 2a, respectively.

In the method of the present invention, the flow of sample breathing gases in sampling line 15a is stopped at a point in the respiratory cycle of the subject when the composition of the breathing gases at the entry to sample line 15a is known. For example, a concentration of CO₂ is known to be present in expiratory breathing gases of the subject but is absent or minimal in the inspiratory breathing gases. See Fig. 4a. In the example shown in the left hand portion of Fig. 4, the breathing gas sample is stopped during expiration by the subject at time 26a. From Figs. 4a and 4b, which show breathing gas flow and concentration characteristics at sampling point 13, it is known that at this time the breathing gases at the entry to sampling line 15a at sampling point 13 will contain the concentration of CO₂ shown by reference number 1 in Fig. 4a.

Fig. 4c shows control signal 23a provided to calibration valve 18 in connection line 18a to energize the valve to block sampling lines 15a, 15b and terminate the flow of the breathing gas sampling 24 in the sampling lines, as shown in Fig. 4d and as would be sensed by flow sensor 9. At time 26a at least the initial portions of sample line 15a will be filled with the high CO₂ concentration breathing gases 1b characteristic of the expiratory phase of the respiratory cycle in which the breathing gas sample flow is stopped.

Valve 18 is controlled to remain closed for a period of time sufficient to permit breathing gases of a different composition to be present in breathing gas passage 11 at the entry to sampling line 15a. Thus, as shown in Figs. 4a and 4b, at time 26b, inspiratory breathing gases will be passing sampling point 13 and the entry to sampling line 15a. These breathing gases 1a will contain little or no CO₂. Time 26b is selected to be sufficiently far into the inspiratory phase of the respiratory cycle, i.e. after the zero crossing between the expiratory and inspiratory phases, so that the breathing gases in the dead space in patient limb 11 upstream of sampling point 13 will have passed the sensor.

At time 26b, signal 23a to valve 18 is terminated and the valve is opened to allow sample breathing gases to again flow in sampling lines 15a, 15b. It will be appreciated that as the breathing gas sample now moves through the sampling lines, it will contain a boundary, or front, between the high CO₂ concentration breathing gases 1b in the sampling line when valve 18 was closed at time 26a and the low or no CO₂ concentration breathing gases 1a entering the sample line when valve 18a is opened at time 26b. This boundary moves through sampling line 15a as the breathing gas sample is drawn through the sampling lines by pump 14. The time it takes this boundary to pass through sampling line 15a from the entry of sampling line 15a at sampling point 13 to gas sensor 16 represents the breathing gas sample transit time through the sampling line and the time delay that must be compensated for in order to synchronize the gas flow and gas concentration measurements so as to obtain accurate measurements of the gas exchange condition of the subject.

To this end, when valve 18 is opened, a calibration value 25, representative of the time delay is reset to zero 25a at time 26b and a timer in measurement and control device 20 is started. Gas sensor 16 analyzes the CO₂ concentration of the breathing gas sample passing through the sensor as shown in Fig. 4e. The sensor will first identify the nature of the breathing gas sample present in sample line 15 when valve 18 was closed at time 26a. In the example given above, the initial nature of the breathing gas sample is one of the high CO₂ concentration breathing gases 1b shown in Fig. 4a. As the flow of the breathing gas sample through gas concentration sensor 16 resumes this will appear as CO₂ concentration measurement 21e.

The gas concentration sensor then, in effect, searches for the boundary between the initial high CO₂ concentration breathing gases and the subsequent low CO₂ concentration breathing gases that started down sampling line 15a, 15b at time 26b. The presence of this boundary in gas sensor 16 will be established when the CO₂ concentration has fallen to a low level of the CO₂ concentration characterizing the breathing gases behind the boundary. In the example shown in Fig. 4, this will occur at time 26c.

The time interval 26b to 26c represents the time it took for the breathing gas sample boundary to pass down sample line 15a from the entry of sample line 15a at sampling point 13 to sensor 16 and is thus the time delay between the gas flow and gas concentration measurements. At time 26c, the timing function is terminated. Fig. 4f shows use of a ramp function in the timer to provide a signal level 25c that is representative of the time delay and may be provided in connection line 20a for use in gas exchange activity determining instrumentation to synchronize the measurements of breathing gas flow and concentration.

With the transit time delay so determined, the normal operation of gas flow sensor 6 and gas concentration sensor 16 resumes until the next time it is desired to determine the breathing gas sample transit time in the sampling line between breathing gas passage 11 and gas concentration sensor 16. The transit time can be periodically remeasured and a new value provided to the gas exchange activity determining instrumentation.

Gas concentration sensor 16 may detect the boundary between the expiratory and inspiratory gases by various techniques. As shown in Fig. 4e, one technique that may be applied is to identify the boundary as present when the measurement of the CO₂ concentration falls to 50 percent of its maximum value. Another way to indicate the boundary as present and to identify time 26c is when the sensed CO₂ concentration reaches its maximum rate of change with respect to time, i.e. its maximum derivative. Other techniques may be used to identify the presence of the boundary between the expiratory and inspiratory gases in the sample line to carry out the method of the present invention.

Instead of, or in addition to, the sample transit time delay, the technique of the present invention may also be used to determine the volume of the sampling line. For this purpose, flow sensor 19 is used. By measuring and integrating the sampling line flow rate over the time interval 26b - 26c, the total sampling line volume can be determined. Determination of the sampling line volume is useful in cases in which the breathing gas sample flow rate varies in the sampling line due, for example, to the operation of ventilator 4. Knowing the sampling line volume and measuring the breathing gas sample flow rate with sensor 19, the needed synchronization between breathing flow and concentration measurements can be calculated.

The technique of the present invention has been described above, in an example in which the sample transit time measurement is initiated at time 26a that occurs in the expiratory phase of the respiratory cycle, then the breathing gas flow direction changes from that of expiration to that of inspiration, and then time 26c occurs in the inspiratory phase of the respiratory cycle. This is in the left-hand side of Fig. 4. The invention may also be practiced in the example shown in the right-hand side of Fig. 4 in which the time 26d at which the flow in the sampling line is stopped occurs during the inspiratory phase of the respiratory cycle, then the direction of breathing gas flow in patient limb 11 changes from that of inspiration to that of expiration, and then time 26e occurs in the expiratory phase of the respiratory cycle. The time interval 26e to 26f represents the sample transit time as indicated by value 25f.

While the invention has been described above as actuating calibration valve 18 to stop the breathing gas flow in sampling line 15a, 15b, it may be equally practiced by controlling the tlow in the sampling line by means of flow generating pump 14. Thus, pump 14 is turned off when flow is not desired in sampling line 15a, 15b and turned on when breathing gas sample flow is desired in the sampling line. Also, total stopping of the flow in sampling line 15a, 15b is not necessary. Rather, the breathing gas sample flow rate in the sampling line may be lessened to a reduced value, for example, 20 percent or less of the usual flow level during, for example, the time between 26a and 26b and 26d and 26e. In these circumstances, however, it is desirable to resume the sampling gas flow at the usual level as promptly as possible after the new gas composition has arrived at sampling point 13, i.e. to shift the time 26b shown in Fig. 4 leftward in the drawing. Delayed resumption to the usual flow level causes a reduced result, since the lower gas flow also drives the boundary towards the sensor 16. In these circumstances, the boundary would therefore be already on its way before the delay time measurement is reset at time 26b or 26e. Furthermore, if the sampling flow is resumed and delay time measurement is reset (26b, 26e) before the gas concentration has changed at the sampling point, the boundary entrance to the sampling line will be delayed in respect to the time 26b, 26e resulting to too long time delay, or too big sampling line volume, determination. Therefore, if the sampling flow is not totally stopped, the resumption has to occur as close to the washout of the dead space in the breathing gas passage as possible.

Alternatively, performing multiple measurements with different assumptions of dead space, the transit delay time or sample line volume can be interpolated from the data.

While Fig. 1 shows ventilator 4 for providing mechanical breathing action to the subject. It will be appreciated that for a spontaneously breathing subject, ventilator 4, limbs 9 and 10, and wye-connector 12 need not be used and could be eliminated when the present invention is practiced.

It is recognized that other equivalents, alternatives, and modifications aside from those expressly stated, are possible and within the scope of the appended claims.

## Claims

1. A method for determining the volume of a breathing gas sampling conduit (15a) extending between a sampling point (13) in a breathing gas passage (11) for the breathing gases of a subject and a gas concentration sensor (16) remote from the sampling point (13), the gas concentration sensor (16) measuring the concentration of a given gas in the sample, the breathing gas alternately flowing in a first direction in the breathing gas passage (11) and in a second, opposite direction in the breathing gas passage (11), the breathing gases exhibiting a first concentration characteristic of the given gas when the breathing gas flow is in the first direction and a second, different concentration characteristic when the breathing gas flow is in the second direction, said method comprising the steps of:
reducing the flow of sample breathing gases in the breathing gas sampling conduit (15a) when the breathing gases are flowing in the breathing gas passage (11) in a first direction and the breathing gases exhibit the concentration characteristic of that breathing gas flow direction;
allowing the flow direction of the breathing gases in the breathing gas passage (11) to reverse to the second direction and the breathing gas concentration characteristic to change to the gas concentration characteristic for the second flow direction;
resuming the flow of sample breathing gases in the breathing gas sampling conduit (15a) and initiating the commencement of a measurement interval;
measuring the breathing gas sample flow rate through the breathing gas sampling conduit (15a);
determining the volume of the breathing gas sampling conduit (15a) using the measured flow rate in the time following resumption of the flow of sample breathing gases;
sensing the concentration of the given gas in the breathing gases sample flowing in the breathing gas sampling conduit (15a) with the breathing gas concentration sensor (16); and
terminating the measurement interval upon the gas concentration sensor (16) sensing an alteration in the given gas concentration characteristic from the characteristic of the first breathing gas flow direction.

2. The method according to claim 1 wherein the initial step is further defined as stopping the breathing gas flow in the breathing gas sampling conduit (15a).

3. The method according to claim wherein the steps of allowing the flow direction to reverse and resuming the flow are such as to cause breathing gases in a dead space in the breathing gas passage upstream of the sampling point (13) to be removed from the sampling point (13).

4. The method according to claim 1 wherein the steps of reducing and resuming the flow of the breathing gas sample in the breathing gas sampling conduit (15a) is carried out by operating a valve.

5. The method according, to claim 1 wherein the steps of reducing and resuming the flow of the breathing gas sample in the breathing gas sampling conduit (15a) is carried out by controlling a breathing gas sample flow generator for the breathing gas sampling conduit (15a).

6. The method according to claim 1 wherein the step of terminating the measurement interval is further defined as terminating the timing interval when there is a change of a predetermined amount in the sensed concentration of the given gas in the breathing gas sample.

7. The method according to claim 1 wherein the step of terminating the measurement interval is further defined as terminating the timing interval when there is a predetermined rate of change in the sensed concentration of the given gas in the breathing gas sample

8. The method according to claim 7 wherein the step of terminating the measurement interval is further defined as terminating the timing interval at the point of maximum rate of change in the sensed concentration of the given gas in the breathing gas sample.

9. The method according to claim 1 further defined as a method for determining the transit time of a breathing gas sample along the breathing gas sampling conduit (15a), said method comprising the additional steps of measuring the gas flow rate through the breathing gas sampling conduit (15a) and using the measured sample breathing gas flow rate and the volume of the breathing gas sampling conduit (15a) to determine the transit time.

10. The method according to any one of claim 1 to 8 further defined as a method for determining the transit time of a breathing gas sample along the breathing gas sampling conduit (15a), said method comprising the additional steps of: initiating the commencement of a transit time timing interval upon the resumption of the flow of sample breathing gases in the breathing gas sampling conduit (15a); and terminating the timing interval upon the gas concentration sensor (16) sensing an alteration in the given gas concentration characteristic from the characteristic of the first breathing gas flow direction to the characteristic of the second breathing gas flow direction, the time interval so defined being an indication of the transit time of the breathing gas sample along the breathing gas sampling conduit extending from the entry of the sampling conduit (15a) to the gas concentration sensor (16).

11. The method according to claim 9 or 10 further including the step of using the transit time so measured to synchronize a gas flow measurement obtained at the sampling point (13) with a given gas concentration measurement obtained by the gas concentration sensor (16).

12. The method according to claim 1 wherein the given gas is CO₂.

13. The method according to claim 1 wherein the given gas is O₂.

14. Apparatus for use with a breathing gas sampling conduit (15a) extending between a sampling point (13) in a breathing gas passage (11) for the breathing gases of a subject and a gas concentration sensor (16) remote from the sampling point (13), the gas concentration sensor measuring the concentration of a given gas in the sample, the breathing gas alternately flowing in a first direction in the breathing gas passage (11) and in a second, opposite direction in the breathing gas passage (11), the breathing gases exhibiting a first concentration characteristic of the given gas when the breathing gas flow is in the first direction and a second, different concentration characteristic when the breathing gas flow is in the second direction, said apparatus comprising:
a measurement and control means (20) coupled to the gas concentration sensor (16);
a breathing gas sensor (6) for determining the flow direction of the breathing gases in the breathing gas passage (11), said breathing gas sensor (16) being coupled to said measurement and control means (20);
flow control means (14, 18) operable by said measurement and control means (20) for controlling the flow of sample breathing gases in the breathing gas sampling conduit (15a), said measurement and control means (20) operating said flow control means (14, 18) to reduce the flow of sample breathing gases in the breathing gas sampling conduit (15a) when the breathing gases are flowing in the breathing gas passage (11) in a first direction and the breathing gases exhibit the concentration characteristic of that breathing gas flow direction, and to thereafter resume the flow of sample breathing gases in the breathing gas sampling conduit (15a) when the flow direction of the breathing gases in the breathing gas passage has reversed to a second direction and the breathing gas concentration characteristic has changed to that of the second direction; and
said measurement and control means (20) having a timer that initiates a measurement interval when the flow of sample breathing gas resumes and terminates the interval when the gas concentration sensor (16) senses an alteration in the given gas concentration characteristic from the characteristic of the first breathing gas flow direction to the characteristic of the second breathing gas flow direction.

15. The apparatus according to claim 14 further defined as one for determining the transit time of a breathing gas sample along a breathing gas sampling conduit (15a) and wherein the measurement interval is an indication of the transit time.

16. The apparatus according to claim 14 further defined as one for determining the volume of the breathing gas sampling conduit (15a), wherein the apparatus further includes a flow sensor (19) for measuring the breathing gas sample flow in said breathing gas sampling conduit (15a) and said measurement and control means (20) is further defined as determining the breathing gas sampling conduit volume from the measured breathing gas sample flow rate and the measurement interval.

17. The apparatus according to claim 16 wherein said measurement and control means (20) is further defined as using the determined conduit volume and the breathing gas sample flow measured by the flow sensor (19) to determine the transit time of a breathing gas sample in the breathing gas sampling conduit (15a).

18. The apparatus according to claim 14 wherein said flow control means comprises a valve (18).

19. The apparatus according to claim 14 wherein said flow control means comprises a sample breathing gas flow generator (14).

20. The apparatus according to claim 14 wherein said flow generator comprises a pump (14).

21. The apparatus according to claim 14 wherein said measurement and control means (20) includes means for determining when there is a change of a predetermined amount in the sensed concentration of the given gas in the breathing gas sample for terminating the timing interval.

22. The apparatus according to claim 14 wherein said measurement and control means (20) includes means for determining when there is a predetermined rate of change in the sensed concentration of the given gas in the breathing gas sample for terminating the timing interval.

23. The apparatus according to claim 14 further including the gas concentration sensor (16) as an element of said apparatus.

24. The apparatus according to claim 14 further including the breathing gas sampling conduit (15a) as an element of said apparatus.

## Patentansprüche

1. Ein Verfahren zum Ermitteln des Volumens einer Atmungsgas-Probenahmeleitung (15a), die sich zwischen einem Probenahmepunkt (13) in einem Atmungsgasdurchgang (11) für die Atmungsgase eines Subjekts und einem fern von dem Probenahmepunkt (13) angeordneten Gaskonzentrationssensor (16) erstreckt, wobei der Gaskonzentrationssensor (16) die Konzentration eines gegebenen Gases in der Probe misst, wobei das Atmungsgas abwechselnd in einer ersten Richtung in dem Atmungsgasdurchgang (11) und in einer zweiten, entgegengesetzten Richtung in dem Atmungsgasdurchgang (11) strömt, wobei die Atmungsgase eine erste Konzentrationcharakteristik des gegebenen Gases aufweisen, wenn der Atmungsgasstrom in der ersten Richtung erfolgt, und eine zweite, unterschiedliche Konzentrationscharakteristik aufweisen, wenn der Atmungsgasstrom in der zweiten Richtung erfolgt, wobei das Verfahren folgende Schritte aufweist:
Reduzieren des Stroms von Probenatmungsgasen in der Atmungsgas-Probenahmeleitung (15a), wenn die Atmungsgase in dem Atmungsgasdurchgang (11) in einer ersten Richtung strömen und die Atmungsgase die Konzentrationscharakteristik dieser Atmungsgas-Strömungsrichtung aufweisen;
Ermöglichen, dass sich die Strömungsrichtung der Atmungsgase in dem Atmungsgasdurchgang (11) zu der zweiten Richtung umkehrt und dass sich die Atmungsgaskonzentrationscharakteristik zu der Gaskonzentrationscharakteristik für die zweite Strömungsrichtung ändert;
Wiederaufnehmen des Strömens von Probenatmungsgasen in der Atmungsgas-Probenahmeleitung (15a) und Einleiten des Beginns eines Messintervalls;
Messen der Atmungsgas-Probeströmungsrate durch die Atmungsgas-Probenahmeleitung (15a);
Ermitteln des Volumens der Atmungsgas-Probenahmeleitung (15a) unter Verwendung der gemessenen Strömungsrate in der Zeit, die auf eine Wiederaufnahme des Strömens von Probenatmungsgasen folgt;
Erfassen der Konzentration des gegebenen Gases in der Atmungsgasprobe, die in der Atmungsgas-Probenahmeleitung (15a) strömt, mit dem Atmungsgaskonzentrationssensor (16); und
Beenden des Messintervalls, nachdem der Gaskonzentrationssensor (16) eine Veränderung der gegebenen Gaskonzentrationscharakteristik von der Charakteristik der ersten Atmungsgasströmungsrichtung erfasst.

2. Das Verfahren gemäß Anspruch 1, bei dem der anfängliche Schritt ferner als Anhalten des Atmungsgasstroms in der Atmungsgas-Probenahmeleitung (15a) definiert ist.

3. Das Verfahren gemäß Anspruch 1, bei dem die Schritte des Ermöglichens, dass sich die Strömungsrichtung umkehrt, und des Wiederaufnehmens des Strömens derart erfolgen, um zu bewirken, dass Atmungsgase, die sich in einem Totraum in dem Atmungsgasdurchgang in Flussrichtung vor dem Probenahmepunkt (13) befinden, von dem Probenahmepunkt (13) entfernt werden.

4. Das Verfahren gemäß Anspruch 1, bei dem die Schritte des Reduzierens und des Wiederaufnehmens des Strömens der Atmungsgasprobe in der Atmungsgas-Probenahmeleitung (15a) durch ein Bedienen eines Ventils durchgeführt werden.

5. Das Verfahren gemäß Anspruch 1, bei dem die Schritte des Verringerns und des Wiederaufnehmens des Strömens der Atmungsgasprobe in der Atmungsgas-Probenahmeleitung (15a) dadurch ausgeführt werden, dass eine Atmungsgasprobenströmungserzeugungsvorrichtung für die Atmungsgas-Probenahmeleitung (15a) gesteuert wird.

6. Das Verfahren gemäß Anspruch 1, bei dem der Schritt des Beendens des Messintervalls ferner als ein Beenden des Zeitgebungsintervalls, wenn bei der erfassten Konzentration des gegebenen Gases in der Atmungsgasprobe eine Änderung einer vorbestimmten Menge vorliegt, definiert ist.

7. Das Verfahren gemäß Anspruch 1, bei dem der Schritt des Beendens des Messintervalls ferner als ein Beenden des Zeitgebungsintervalls, wenn eine vorbestimmte Änderungsrate der erfassten Konzentration des gegebenen Gases in der Atmungsgasprobe vorliegt, definiert ist.

8. Das Verfahren gemäß Anspruch 7, bei dem der Schritt des Beendens des Messintervalls ferner als ein Beenden des Zeitgebungsintervalls an dem Punkt einer maximalen Änderungsrate der erfassten Konzentration des gegebenen Gases in der Atmungsgasprobe definiert ist.

9. Das Verfahren gemäß Anspruch 1, das ferner als Verfahren zum Ermitteln der Durchlaufzeit einer Atmungsgasprobe entlang der Atmungsgas-Probenahmeleitung (15a) definiert ist, wobei das Verfahren die zusätzlichen Schritte des Messens der Gasströmungsrate durch die Atmungsgas-Probenahmeleitung (15a) und des Verwendens der gemessenen Probenatmungsgasströmungsrate und des Volumens der Atmungsgas-Probenahmeleitung (15a), um die Durchlaufzeit zu ermitteln, umfasst.

10. Das Verfahren gemäß einem der Ansprüche 1 bis 8, das ferner als Verfahren zum Ermitteln der Durchlaufzeit einer Atmungsgasprobe entlang der Atmungsgas-Probenahmeleitung (15a) definiert ist, wobei das Verfahren die folgenden zusätzlichen Schritte aufweist: Einleiten des Beginns eines Durchlaufzeit-Zeitgebungsintervalls auf die Wiederaufnahme des Strömens von. Probenatmungsgasen in der Atmungsgas-Probenahmeleitung (15a) hin; und Beenden des Zeitgebungsintervalls, nachdem der Gaskonzentrationssensor (16) eine Änderung der gegebenen Gaskonzentrationscharakteristik bezüglich der Charakteristik der ersten Atmungsgasströmungsrichtung zu der Charakteristik der zweiten Atmungsgasströmungsrichtung hin erfasst, wobei das auf diese Weise definierte Zeitintervall eine Angabe der Durchlaufzeit der Atmungsgasprobe entlang der Atmungsgas-Probenahmeleitung ist, die sich von dem Eingang der Probenahmeleitung (15a) zu dem Gaskonzentrationssensor (16) erstreckt.

11. Das Verfahren gemäß Anspruch 9 oder 10, das ferner den Schritt des Verwendens der auf diese Weise gemessenen Durchlaufzeit, um eine an dem Probenahmepunkt (13) erhaltene Gasströmungsmessung mit einer durch den Gaskonzentrationssensor (16) erhaltenen Konzentrationsmessung des gegebenen Gases zu synchronisieren.

12. Das Verfahren gemäß Anspruch 1, bei dem das gegebene Gas CO₂ ist.

13. Das Verfahren gemäß Anspruch 1, bei dem das gegebene Gas O₂ ist.

14. Vorrichtung zur Verwendung mit einer Atmungsgas-Probenahmeleitung (15a), die sich zwischen einem Probenahmepunkt (13) in einem Atmungsgasdurchgang (11) für die Atmungsgase eines Subjekts und einem fern von dem Probenahmepunkt (13) angeordneten Gaskonzentrationssensor (16) erstreckt, wobei der Gaskonzentrationssensor die Konzentration eines gegebenen Gases in der Probe misst, wobei das Atmungsgas abwechselnd in einer ersten Richtung in dem Atmungsgasdurchgang (11) und in einer zweiten, entgegengesetzten Richtung in dem Atmungsgasdurchgang (11) strömt, wobei die Atmungsgase eine erste Konzentrationcharakteristik des gegebenen Gases aufweisen, wenn der Atmungsgasstrom in der ersten Richtung erfolgt, und eine zweite, unterschiedliche Konzentrationscharakteristik aufweisen, wenn der Atmungsgasstrom in der zweiten Richtung erfolgt, wobei die Vorrichtung folgende Merkmale aufweist:
eine Mess- und Steuerungseinrichtung (20), die mit dem Gaskonzentrationssensor (16) gekoppelt ist;
einen Atmungsgassensor (6) zum Ermitteln der Strömungsrichtung der Atmungsgase in dem Atmungsgasdurchgang (11), wobei der Atmungsgassensor (16) mit der Mess- und Steuerungseinrichtung (20) gekoppelt ist;
eine Strömungssteuerungseinrichtung (14, 18), die durch die Mess- und Steuerungseinrichtung (20) betreibbar ist, um die Strömung von Probenatmungsgasen in der Atmungsgas-Probenahmeleitung (15a) zu steuern, wobei die Mess- und Steuerungseinrichtung (20) die Strömungssteuerungseinrichtung (14, 18) betreibt, um die Strömung von Probenatmungsgasen in der Atmungsgas-Probenahmeleitung (15a) zu reduzieren, wenn die Atmungsgase in dem Atmungsgasdurchgang (11) in einer ersten Richtung strömen und die Atmungsgase die Konzentrationscharakteristik dieser Atmungsgas-Strömungsrichtung aufweisen, und danach das Strömen der Probenatmungsgase in der Atmungsgas-Probenahmeleitung (15a) wiederaufzunehmen, wenn sich die Strömungsrichtung der Atmungsgase in dem Atmungsgasdurchgang zu einer zweiten Richtung umgekehrt hat und sich die Atmungsgaskonzentrationscharakteristik zu der der zweiten Richtung geändert hat; und
wobei die Mess- und Steuerungseinrichtung (20) einen Zeitgeber aufweist, der ein Messintervall einleitet, wenn das Strömen von Probenatmungsgas wiederaufgenommen wird, und das Intervall beendet, wenn der Gaskonzentrationssensor (16) eine Änderung der Konzentrationscharakteristik des gegebenen Gases bezüglich der Charakteristik der ersten Atmungsgasströmungsrichtung zu der Charakteristik der zweiten Atmungsgasströmungsrichtung erfasst.

15. Die Vorrichtung gemäß Anspruch 14, die ferner als eine zum Ermitteln der Durchlaufzeit einer Atmungsgasprobe entlang einer Atmungsgas-Probenahmeleitung (15a) definiert ist, und bei der das Messintervall eine Angabe der Durchlaufzeit ist.

16. Die Vorrichtung gemäß Anspruch 14, die ferner als eine zum Ermitteln des Volumens der Atmungsgas-Probenahmeleitung (15a) definiert ist, wobei die Vorrichtung ferner einen Strömungssensor (19) zum Messen der Atmungsgasprobenströmung in der Atmungsgas-Probenahmeleitung (15a) umfasst, und die Mess- und Steuerungseinrichtung (20) ferner dazu definiert ist, das Volumen der Atmungsgas-Probenahmeleitung aus der gemessenen AtmungsgasProbeströmungsrate und dem Messintervall zu ermitteln.

17. Die Vorrichtung gemäß Anspruch 16, bei der die Mess- und Steuerungseinrichtung (20) ferner dazu definiert ist, das ermittelte Leitungsvolumen und die durch den Strömungssensor (19) gemessene Atmungsgasprobenströmung dazu zu verwenden, die Durchlaufzeit einer Atmungsgasprobe in der Atmungsgas-Probenahmeleitung (15a) zu ermitteln.

18. Die Vorrichtung gemäß Anspruch 14, bei der die Strömungssteuerungseinrichtung ein Ventil (18) umfasst.

19. Die Vorrichtung gemäß Anspruch 14, bei der die Strömungssteuerungseinrichtung eine Probenatmungsgasströmungserzeugungsvorrichtung (14) umfasst.

20. Die Vorrichtung gemäß Anspruch 14, bei der die Strömungserzeugungsvorrichtung eine Pumpe (14) umfasst.

21. Die Vorrichtung gemäß Anspruch 14, bei der die Mess- und Steuerungseinrichtung (20) eine Einrichtung zum Ermitteln dessen umfasst, wann eine Änderung der erfassten Konzentration des gegebenen Gases in der Atmungsgasprobe um eine vorbestimmte Menge vorliegt, um das Zeitgebungsintervall zu beenden.

22. Die Vorrichtung gemäß Anspruch 14, bei der die Mess- und Steuerungseinrichtung (20) eine Einrichtung zum Ermitteln dessen umfasst, wann bei der erfassten Konzentration des gegebenen Gases in der Atmungsgasprobe eine vorbestimmte Änderungsrate vorliegt, um das Zeitgebungsintervall zu beenden.

23. Die Vorrichtung gemäß Anspruch 14, die ferner den Gaskonzentrationssensor (16) als Element der Vorrichtung umfasst.

24. Die Vorrichtung gemäß Anspruch 14, die ferner die Atmungsgas-Probenahmeleitung (15a) als Element der Vorrichtung umfasst.

## Revendications

1. Procédé de détermination du volume d'une conduite d'échantillonnage de gaz respiratoire (15a) s'étendant entre un point d'échantillonnage (13) dans un passage de gaz respiratoire (11) pour les gaz respiratoires d'un sujet et un capteur de concentration gazeuse (16) à l'écart du point d'échantillonnage (13), le capteur de concentration gazeuse (16) mesurant la concentration d'un gaz donné dans l'échantillon, le gaz respiratoire s'écoulant en alternance dans une première direction dans le passage de gaz respiratoire (11) et dans une seconde direction opposée dans le passage de gaz respiratoire (11), les gaz respiratoires présentant une première caractéristique de concentration du gaz donné lorsque l'écoulement de gaz respiratoire est dans la première direction et une seconde caractéristique de concentration différente lorsque l'écoulement de gaz respiratoire est dans la seconde direction, ledit procédé comprenant les étapes consistant à :
réduire l'écoulement de gaz respiratoires échantillonnés dans la conduite d'échantillonnage de gaz respiratoire (15a) lorsque les gaz respiratoires s'écoulent dans le passage de gaz respiratoire (11) dans une première direction et les gaz respiratoires présentent la caractéristique de concentration de cette direction d'écoulement de gaz respiratoire ;
permettre à la direction d'écoulement des gaz respiratoires dans le passage de gaz respiratoire (11) de retourner à la seconde direction et à la caractéristique de concentration de gaz respiratoire de changer en la caractéristique de concentration gazeuse pour la seconde direction d'écoulement ;
reprendre l'écoulement de gaz respiratoires échantillonnés dans la conduite d'échantillonnage de gaz respiratoire (15a) et amorcer le commencement d'un intervalle de mesure ;
mesurer le débit d'échantillon de gaz respiratoire à travers la conduite d'échantillonnage de gaz respiratoire (15a) ;
déterminer le volume de la conduite d'échantillonnage de gaz respiratoire (15a) en utilisant le débit mesuré dans la durée suivant la reprise de l'écoulement des gaz respiratoires échantillonnés ;
détecter la concentration du gaz donné dans l'échantillon de gaz respiratoires s'écoulant dans la conduite d'échantillonnage de gaz respiratoire (15a) avec le capteur de concentration de gaz respiratoire (16) ; et
terminer l'intervalle de mesure lorsque le capteur de concentration gazeuse (16) détecte une altération de la caractéristique de concentration du gaz donné par rapport à la caractéristique de la première direction d'écoulement de gaz respiratoire.

2. Procédé selon la revendication 1, dans lequel l'étape initiale est définie en outre comme l'arrêt de l'écoulement de gaz respiratoire dans la conduite d'échantillonnage de gaz respiratoire (15a).

3. Procédé selon la revendication 1, dans lequel les étapes consistant à permettre à la direction d'écoulement de s'inverser et à reprendre l'écoulement sont telles qu'elles provoquent le retrait du point d'échantillonnage (13) des gaz respiratoires dans un espace mort dans le passage de gaz respiratoire en amont du point d'échantillonnage (13).

4. Procédé selon la revendication 1, dans lequel les étapes consistant à réduire et reprendre l'écoulement de l'échantillon de gaz respiratoire dans la conduite d'échantillonnage de gaz respiratoire (15a) sont effectuées en opérant une vanne.

5. Procédé selon la revendication 1, dans lequel les étapes consistant à réduire et reprendre l'écoulement de l'échantillon de gaz respiratoire dans la conduite d'échantillonnage de gaz respiratoire (15a) sont effectuées en commandant un générateur d'écoulement d'échantillon de gaz respiratoire pour la conduite d'échantillonnage de gaz respiratoire (15a).

6. Procédé selon la revendication 1, dans lequel l'étape consistant à terminer l'intervalle de mesure est définie en outre comme le fait de terminer l'intervalle de chronométrage lorsqu'il y a un changement d'une quantité prédéterminée de la concentration détectée du gaz donné dans l'échantillon de gaz respiratoire.

7. Procédé selon la revendication 1, dans lequel l'étape consistant à terminer l'intervalle de mesure est définie en outre comme le fait de terminer l'intervalle de chronométrage lorsqu'il y a un taux de changement prédéterminé de la concentration détectée du gaz donné dans l'échantillon de gaz respiratoire.

8. Procédé selon la revendication 7, dans lequel l'étape consistant à terminer l'intervalle de mesure est définie en outre comme le fait de terminer l'intervalle de chronométrage au point de taux de changement maximal de la concentration détectée du gaz donné dans l'échantillon de gaz respiratoire.

9. Procédé selon la revendication 1, défini en outre comme un procédé de détermination du temps de transit d'un échantillon de gaz respiratoire le long de la conduite d'échantillonnage de gaz respiratoire (15a), ledit procédé comprenant les étapes supplémentaires consistant à mesurer le débit gazeux à travers la conduite d'échantillonnage de gaz respiratoire (15a) et utiliser le débit mesuré de gaz respiratoire échantillonné et le volume de la conduite d'échantillonnage de gaz respiratoire (15a) pour déterminer le temps de transit.

10. Procédé selon l'une quelconque des revendications 1 à 8, défini en outre comme un procédé de détermination du temps de transit d'un échantillon de gaz respiratoire le long de la conduite d'échantillonnage de gaz respiratoire (15a), ledit procédé comprenant les étapes supplémentaires consistant à : amorcer le commencement d'un intervalle de chronométrage de temps de transit lors de la reprise de l'écoulement de gaz respiratoires échantillonnés dans la conduite d'échantillonnage de gaz respiratoire (15a) ; et terminer l'intervalle de chronométrage lors de la détection par le capteur de concentration gazeuse (16) d'une altération de la caractéristique de concentration du gaz donné entre la caractéristique de la première direction d'écoulement de gaz respiratoire et la caractéristique de la seconde direction d'écoulement de gaz respiratoire, l'intervalle de temps ainsi défini étant une indication du temps de transit de l'échantillon de gaz respiratoire le long de la conduite d'échantillonnage de gaz respiratoire s'étendant de l'entrée de la conduite d'échantillonnage (15a) au capteur de concentration gazeuse (16).

11. Procédé selon la revendication 9 ou 10, comprenant en outre l'étape consistant à utiliser le temps de transit ainsi mesuré pour synchroniser une mesure d'écoulement gazeux obtenue au niveau du point d'échantillonnage (13) avec une mesure de concentration du gaz donné obtenue par le capteur de concentration gazeuse (16).

12. Procédé selon la revendication 1, dans lequel le gaz donné est du CO₂.

13. Procédé selon la revendication 1, dans lequel le gaz donné est O₂.

14. Appareil destiné à l'utilisation avec une conduite d'échantillonnage de gaz respiratoire (15a) s'étendant entre un point d'échantillonnage (13) dans un passage de gaz respiratoire (11) pour les gaz respiratoires d'un sujet et un capteur de concentration gazeuse (16) à l'écart du point d'échantillonnage (13), le capteur de concentration gazeuse mesurant la concentration d'un gaz donné dans l'échantillon, le gaz respiratoire s'écoulant en alternance dans une première direction dans le passage de gaz respiratoire (11) et dans une seconde direction opposée dans le passage de gaz respiratoire (11), les gaz respiratoires présentant une première caractéristique de concentration du gaz donné lorsque l'écoulement de gaz respiratoire est dans la première direction et une seconde caractéristique de concentration différente lorsque l'écoulement de gaz respiratoire est dans la seconde direction, ledit appareil comprenant :
un moyen de mesure et de commande (20) accouplé au capteur de concentration gazeuse (16) ;
un capteur de gaz respiratoire (6) pour déterminer la direction d'écoulement des gaz respiratoires dans le passage de gaz respiratoire (11), ledit capteur de gaz respiratoire (16) étant accouplé audit moyen de mesure et de commande (20) ;
des moyens de commande d'écoulement (14, 18) pouvant être opérés par ledit moyen de mesure et de commande (20) pour commander l'écoulement de gaz respiratoires échantillonnés dans la conduite d'échantillonnage de gaz respiratoire (15a), ledit moyen de mesure et de commande (20) opérant lesdits moyens de commande d'écoulement (14, 18) pour réduire l'écoulement de gaz respiratoires échantillonnés dans la conduite d'échantillonnage de gaz respiratoire (15a) lorsque les gaz respiratoires s'écoulent dans le passage de gaz respiratoire (11) dans une première direction et les gaz respiratoires présentent la caractéristique de concentration de cette direction d'écoulement de gaz respiratoire, et pour reprendre ensuite l'écoulement de gaz respiratoires échantillonnés dans la conduite d'échantillonnage de gaz respiratoire (15a) lorsque la direction d'écoulement des gaz respiratoires dans le passage de gaz respiratoire est retournée à une seconde direction et la caractéristique de concentration de gaz respiratoire a changé en celle de la seconde direction ; et
ledit moyen de mesure et de commande (20) ayant une minuterie qui amorce un intervalle de mesure lorsque l'écoulement de gaz respiratoire échantillonné reprend et termine l'intervalle lorsque le capteur de concentration gazeuse (16) détecte une altération de la caractéristique de concentration du gaz donné entre la caractéristique de la première direction d'écoulement de gaz respiratoire et la caractéristique de la seconde direction d'écoulement de gaz respiratoire.

15. Appareil selon la revendication 14, défini en outre comme un appareil pour déterminer le temps de transit d'un échantillon de gaz respiratoire le long d'une conduite d'échantillonnage de gaz respiratoire (15a) et dans lequel l'intervalle de mesure est une indication du temps de transit.

16. Appareil selon la revendication 14, défini en outre comme un appareil pour déterminer le volume de la conduite d'échantillonnage de gaz respiratoire (15a), dans lequel l'appareil comprend en outre un capteur d'écoulement (19) pour mesurer l'écoulement d'échantillon de gaz respiratoire dans ladite conduite d'échantillonnage de gaz respiratoire (15a) et ledit moyen de mesure et de commande (20) est défini en outre comme déterminant le volume de la conduite d'échantillonnage de gaz respiratoire à partir du débit mesuré d'échantillon de gaz respiratoire et de l'intervalle de mesure.

17. Appareil selon la revendication 16, dans lequel ledit moyen de mesure et de commande (20) est défini en outre comme utilisant le volume de conduite déterminé et l'écoulement d'échantillon de gaz respiratoire mesuré par le capteur d'écoulement (19) pour déterminer le temps de transit d'un échantillon de gaz respiratoire dans la conduite d'échantillonnage de gaz respiratoire (15a).

18. Appareil selon la revendication 14, dans lequel ledit moyen de commande d'écoulement comprend une vanne (18).

19. Appareil selon la revendication 14, dans lequel ledit moyen de commande d'écoulement comprend un générateur d'écoulement de gaz respiratoire échantillonné (14).

20. Appareil selon la revendication 14, dans lequel ledit générateur d'écoulement comprend une pompe (14).

21. Appareil selon la revendication 14, dans lequel ledit moyen de mesure et de commande (20) comprend des moyens pour déterminer lorsqu'il y a un changement d'une quantité prédéterminée de la concentration détectée du gaz donné dans l'échantillon de gaz respiratoire pour terminer l'intervalle de temps.

22. Appareil selon la revendication 14, dans lequel ledit moyen de mesure et de commande (20) comprend des moyens pour déterminer lorsqu'il y a un taux de changement prédéterminé de la concentration détectée du gaz donné dans l'échantillon de gaz respiratoire pour terminer l'intervalle de temps.

23. Appareil selon la revendication 14, comprenant en outre le capteur de concentration gazeuse (16) en tant qu'élément dudit appareil.

24. Appareil selon la revendication 14, comprenant en outre la conduite d'échantillonnage de gaz respiratoire (15a) en tant qu'élément dudit appareil.
